Europäisches Patentamt

European Patent Office

Office européen des brevets

(19)

(11) Veröffentlichungsnummer: **0 248 114 B1**

(12) **EUROPÄISCHE PATENTSCHRIFT**

(45) Veröffentlichungstag der Patentschrift: 08.05.91

(51) Int. Cl.⁵: **C10G 21/20**, C10G 7/08

(21) Anmeldenummer: **86116199.0**

(22) Anmeldetag: **21.11.86**

(54) Verfahren zur Abtrennung von Aromaten aus Kohlenwasserstoffgemischen beliebigen Aromatengehaltes.

(30) Priorität: 25.01.86 DE 3602240

(43) Veröffentlichungstag der Anmeldung:
09.12.87 Patentblatt 87/50

(45) Bekanntmachung des Hinweises auf die
Patenterteilung:
08.05.91 Patentblatt 91/19

(84) Benannte Vertragsstaaten:
**AT BE DE ES FR GB IT LU NL SE**

(56) Entgegenhaltungen:
**DE-A- 2 035 771**
**DE-B- 1 568 940**
**FR-A- 1 470 472**
**FR-A- 2 209 592**

(73) Patentinhaber: **Krupp Koppers GmbH**
**Altendorfer Strasse 120**
**W-4300 Essen 1(DE)**

(72) Erfinder: **Emmrich, Gerd, Dipl.-Ing.**
**Kamperfeld 21**
**W-43oo Essen 1(DE)**
Erfinder: **Preusser, Gerhard, Dr., Dipl.-Chem.**
**Lönsberg 24**
**W-43oo Esssen 1(DE)**

## Beschreibung

Die Erfindung betrifft ein Verfahren zur Abtrennung von Aromaten aus Kohlenwasserstoffgemischen beliebigen Aromatengehaltes unter Verwendung von N-substituierten Morpholinen, deren Substituenten nicht mehr als 7 C-Atome aufweisen, als selektives Lösungsmittel, wobei nur ein Teil der erforderlichen Gesamtlösungsmittelmenge auf den obersten Boden der Extraktivdestillationskolonne aufgegeben wird.

Das vorstehend beschriebene Aromatengewinnungsverfahren ist bereits seit einer ganzen Reihe von Jahren bekannt und hat sich, insbesondere unter Verwendung von N-Formylmorpholin als selektivem Lösungsmittel in der Zwischenzeit in der Praxis auf verschiedenen großtechnischen Anlagen gut bewährt. Wie aus der DE-C- 15 68 94o hervorgeht, wird hierbei normalerweise das aus der Extraktivdestillationskolonne abgezogene Sumpfprodukt in eine nachgeschaltete Abtreiberkolonne eingeleitet, in der die in ihm als Extrakt enthaltenen Aromaten destillativ vom Lösungsmittel abgetrennt werden. Das Lösungsmittel wird sodann aus dem Sumpf der Abtreiberkolonne abgezogen und zur Wiederverwendung in die Extraktivdestillationskolonne zurückgeführt. Hierbei erfolgt die Einleitung bzw. Wiedereinleitung des Lösungsmittels in der Regel auf den obersten Boden der Extraktivdestillationskolonne. Die nichtaromatischen Kohlenwasserstoffe werden dagegen über Kopf aus der Extraktivdestillationskolonne abgezogen und können in nachgeschalteten Kondensationseinrichtungen ganz oder teilweise kondensiert werden. Aus der DE-C-20 35 771 ist ferner eine Variante dieser Arbeitsweise bekannt, bei der nur ein Teil des selektiven Lösungsmittels auf den obersten Boden der Extraktivdestillationskolonne aufgegeben wird, während der Rest des Lösungmittels gemeinsam mit dem Einsatzprodukt durch die für die Zuführung des Einsatzproduktes vorgesehene Leitung in die Extraktivdestillationskolonne eingeleitet wird.

Eine Aufteilung der Gesamtlösungsmittelmenge in zwei Teilströme ist auch bei dem in der FR-A-2 209 592 beschriebenen Extraktivdestillationsverfahren zur Trennung von im gleichen Temperaturbereich siedenden Stoffen verschi edener chemischer Struktur vorgesehen. Hierbei sollen beide Tei lströme oberhalb des Aufgabebodens für das Einsatzprodukt in die Extraktivdestillationskolonne eingeleitet werden, wobei der auf einen der oberen Böden aufgegebene Teilstrom eine niedrigere Temperatur aufweisen soll als der darunter aufgegebene Tei lstrom. In den Ausführungsbei spielen wird dabei die Aufarbeitung von hydriertem Kokereibenzol und vollhydriertem Pyrolysebenzin unter Verwendung von N-Methylpyrrolidon als selektivem Lösungsmittel beschrieben.

Obwohl sich das weiter oben beschriebene Verfahren der eingangs genannten Art - wie bereits festgestellt wurde - in der Praxis gut bewährt hat, ist man doch bestrebt, diese Arbeitsweise noch weiter zu optimieren, und zwar insbesondere im Hinblick auf eine Erhöhung der Durchsatzkapazi tät und eine ruhigere Fahrweise der Extraktivdestillationskolonne sowie eine Verbesserung des Trennergebnisses.

In Verfolgung dieser Aufgabe wurde nun überraschenderweise gefunden, daß bei einem Verfahren der eingangs genannten Art eine derartige Optimierung erreicht werden kann, wenn erfindungsgemäß der Teil der Gesamt lösungs-mittelmenge, der nicht auf den obersten Boden der Extraktivdestillationskolonne aufgegeben wird, zwischen 10 und 40 Gew.-% beträgt, wobei dieser Teil in mindestens zwei Teilströmen aufgeteilt und in Abhängigkeit vom Temperaturverlauf in der Extraktivdestillationskolonne auf Böden oberhalb der Einsatzproduktaufgabe in der Weise in die Extraktivdestillationskolonne eingeleitet wird, daß die Menge des über die einzelnen Teilströme zugeführten Lösungsmittels in Richtung auf den obersten Boden von Stufe zu Stufe abnimmt und wobei ferner die Aufgabetemperatur des jeweiligen Teilstromes so eingestellt wird, daß sie die Temperatur auf den entsprechenden Aufgabeboden nicht übersteigt und auch nicht um mehr als 10° C unterschreitet.

Der Erfindung liegt dabei die Erkenntnis zugrunde, daß diese Optimierung umso wirkungsvoller ist, je mehr die Lösungsmittelaufgabe dem konzentrationsabhängigen Temperaturverlauf in der Extraktivdestillationskolonne angepaßt wird.

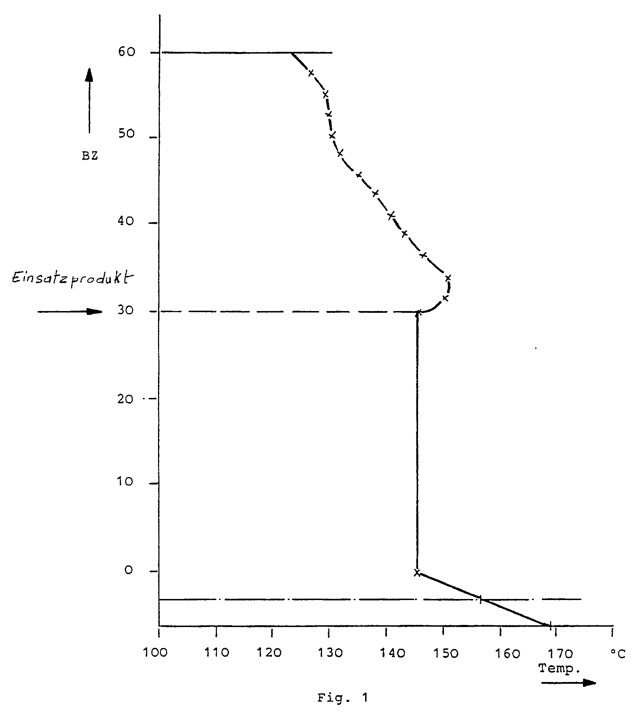

In Fig. 1 ist als Beispiel ein derartiger Temperaturverlauf für eine mit 60 Böden versehene Extraktivdestillatilonskolonne graphisch dargestellt. Als Einsatzprodukt diente die Benzolfraktion eines Pyrolysebenzins und als Lösungsmittel N-Formylmorpholin. Auf der Abszisse ist die Temperatur (Temp.) und auf der Ordiante die Bodenzahl (Bz.) angegeben. Die Aufgabe des Einsatzproduktes erfolgt in diesem Falle auf den 30. Boden. Der Kurvenverlauf zeigt, daß sich im Bereich von der Einsatzproduktaufgabe bis zum Sumpf der Kolonne die Aufkonzentrierung der Aromaten im Extrakt kaum in einer Temperaturerhöhung bemerkbar macht, obwohl dort das höchste molare Verhältnis von Lösungsmittel zu Ei nsatzkoh lenwasserstoff erforderlich ist, da dort die Dampfdrücke der Nichtaromaten gegenüber den Aromaten am weitesten auseinander liegen müssen, um auch kleinste Nichtaromatenmengen aus der Lösung auszutreiben. Erst in den beiden Verdampfungsstufen unmittelbar über dem Sumpf der Extraktivdestillationskolonne steigt die Temperatur in zwei Stufen sprungartig an. Oberhalb der Einsatzproduktaufgabe zeigt der Verlauf der Temperaturkurve einen deutlichen Sprung. Dies ist damit zu erklären, daß hier zunächst sehr viel

EP 0 248 114 B1

Aromatendämpfe in Lösung gehen und ihre Kondensationswärme an das Lösungsmittel abgeben. Erst danach nimmt von Boden zu Boden die in Lösung gehende Aromatendämpfemenge stetig ab, und der Temperaturverlauf zeigt bis zum obersten Boden der Extraktivdestillationskolonne hin eine fallende Tendenz.

Das erfindungsgemäße Verfahren nutzt diesen Temperaturverlauf in der Extraktivdestillationskolonne, in dem es eine Aufgabe des Lösungsmittelteilstromes, der nicht auf den obersten Boden der Extraktivdestillationskolonne aufgegeben werden soll, in mindestens zwei Teilströmen auf Böden oberhalb der Einsatzproduktaufgabe vorsieht. Hierbei ist es von entscheidender Bedeutung, daß sich die Aufgabetemperatur des Lösungsmittels jeweils am Temperaturverlauf in der Kolonne orientiert. Das heißt, diese Temperatur darf die Temperatur auf dem entsprechenden Aufgabeboden nicht übersteigen, da sonst bereits im Lösungsmittel gelöste Aromaten wieder ausgetrieben würden. Allenfalls ist es zulässig, daß die Aufgabetemperatur des Lösungsmittels die Temperatur auf dem Aufgabeboden geringfügig, d.h. um nicht mehr als 1o °C unterschreitet.

Die Lage der Aufgabestellen der einzelnen Teilströme des Lösungsmittels, die zwischen der Einsatzproduktaufgabe und dem obersten Boden der Extraktivdestillationskolonne in diese Kolonne eingeleitet werden sollen, orientiert sich dabei ebenfalls am Temperaturverlauf in dieser Kolonne. Die Aufgabe der einzelnen Teilströme soll nämlich insbesondere in dem Bereich erfolgen, in dem der Temperaturverlauf oberhalb der Einsatzproduktaufgabe einen deutlichen Sprung aufweist. In der Praxis hängt das natürlich von der Konstruktion und den konkreten Betriebsbedingungen der jeweiligen Extraktivdestillationskolonne ab. In der Regel wird man jedoch davon ausgehen können, daß die Aufgabe der einzelnen Teilströme vorzugsweise im Bereich zwischen dem dritten und zehnten Boden oberhalb der Einsatzproduktaufgabe erfolgen sollte.

Wie aus dem in Fig. 1 dargestellten Temperaturverlauf ersichtlich ist, wird vom Einsatzproduktaufgabeboden bis zum Kopf der Extraktivdestillationskolonne hin der Aromatengehalt kontinuierlich abgebaut. Das bedeutet aber, daß zum Lösen der jeweiligen Restmenge an Aromaten eine immer geringere Menge an Lösungsmittel erforderlich ist. Da sich die ideale Verteilung der Lösungsmittelmenge im Bereich zwischen der Einsatzproduktaufgabe und dem obersten Boden der Extraktivdestillationskolonne an diesen Verhältnissen orientieren sollte, ist der Grad der Optimierung der Extraktivdestillation umso größer, je höher die Anzahl der Aufgabestellen des Lösungsmittels ist, wobei die Menge des aufgegebenen Lösungsmittels entsprechend dem tatsächlichen Bedarf von Stufe zu Stufe abnehmen kann.

In der Praxis wird die Anzahl der Aufgabestellen vorzugsweise zwischen zwei und fünf liegen. Sie ist einerseits abhängig von der Zusammensetzung des Einsatzproduktes, wobei ein Einsatzprodukt mit hohem Nichtaromatengehalt, wie z.B. Pyrolysebenzin, mehr Aufgabestellen erfordert als ein Einsatzprodukt mit niedrigem Aromatengehalt, wie z.B. Kokereibenzol. Andererseits ist es aus wirtschaftlichen Gründen nur bei großen Durchsatzkapazitäten der Extraktivdestillationskolonne sinnvoll, mehr als zwei Aufgabestellen vorzusehen, um den erforderlichen Aufwand an meß- und regeltechnischen Einrichtungen in vertretbaren Grenzen zu halten. Generell läßt sich sagen, je größer die Kapazität einer Extraktivdestillationskolonne ist, umso wirkungsvoller ist eine möglichst nahe Anpassung der Lösungsmittelaufgabe an den Temperaturverlauf in der Kolonne. Je besser diese Anpassung vorgenommen werden soll, desto größer muß allerdings die Anzahl der geregelten Aufgabestellen für das Lösungsmittel sein.

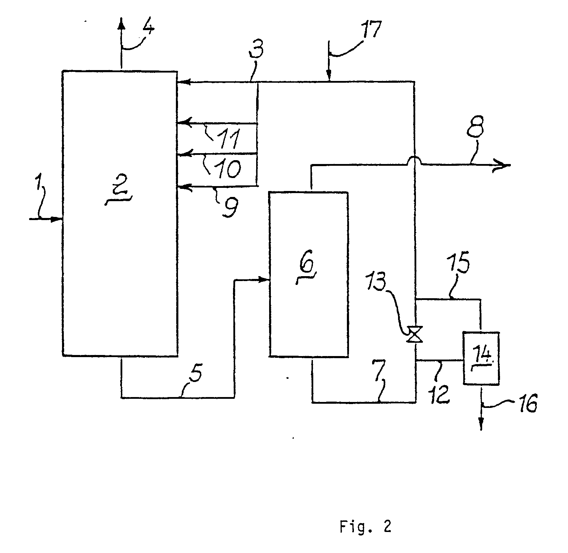

Das bei der Durchführung des erfindungsgemäßen Verfahrens zur Anwendung gelangende Verfahrensschema ist das bei derartigen Extraktivdestillationen übliche Verfahrensschema, das in Fig. 2 als Fließschema in vereinfachter Form dargestellt ist. Hierbei wird das aufzutrennende Kohlenwasserstoffgemisch, das als Einsatzprodukt dient, durch die Leitung 1 in den mittleren Teil der mit Böden oder ähnlichen Einbauten versehenen Extraktivdestillationskolonne 2 eingeleitet. Das Einsatzprodukt ist dabei entweder vor dem Eintritt in die Extraktivdestillationskolonne 2 auf Temperaturen dicht unterhalb des Siedepunktes erhitzt worden, oder es wird bereits im teilverdampften Zustand in die Extraktivdestillationskolonne 2 eingeführt. Durch die Leitung 3 wird der Teil des selektiven Lösungsmittels in die Extraktivdestillationskolonne 2 eingeleitet, der auf den obersten Boden dieser Kolonne aufgegeben wird. Durch die Leitungen 9 bis 11 wird jener Teil des Lösungsmittels in die Extraktivdestillationskolonne 2 eingeleitet, der erfindungsgemäß oberhalb der Einsatzproduktaufgabe in die Kolonne eingeleitet werden soll. Die im Fließschema dargestellte Zahl von drei Aufgabestellen dient dabei nur als Beispiel. Das aufgegebene Lösungsmittel fließt über die Einbauten der Extraktivdestillationskolonne 2 herab nach unten, wobei es die dampfförmigen Aromaten aufnimmt. Die nichtaromatischen Kohlenwasserstoffe entweichen durch Leitung 4 am Kopf der Kolonne und können in einer im Fließschema nicht dargestellten Kondensationseinrichtung kondensiert werden. Das flüssige Sumpfprodukt der Extraktivdestillationskolonne (Extrakt) besteht aus dem selektiven Lösungsmittel und den darin gelösten Aromaten und wird durch die Leitung 5 aus der Extraktivdestillationskolonne 2 abgezogen und gelangt in die Kolonne 6, in der die Aromaten destillativ vom selektiven Lösungsmittel abgetrennt werden. Das selektive Lösungsmittel wird durch die Leitung 7 aus dem Kolonnensumpf entfernt

3

und gelangt über die Leitung 3 bzw. die Leitungen 9 bis 11 wieder in die Extraktivdestillationskolonne 2 zurück, während die Aromatendämpfe über die Leitung 8 über Kopf aus der Kolonne 6 entweichen und in nicht dargestellten Kondensationseinrichtungen kondensiert werden und danach gegebenenfalls weiter aufgetrennt werden können.

Da sich im Laufe der Zeit im selektiven Lösungsmittel Verunreinigungen anreichern können, ist im Bereich der Leitung 7 die Abzweigleitung 12 vorgesehen, durch die bei entsprechender Stellung des Ventils 13 eine Teilmenge des selektiven Lösungsmittels zur Regenerierungseinrichtung 14 gelangen kann. Das regenerierte Lösungsmittel wird durch die Leitung 15 wieder in den Kreislauf (Leitung 3) zurückgeführt, während die ausgeschiedenen Verunreinigungen durch die Leitung 16 aus der Regeneriereinrichtung 14 abgezogen werden. Die Leitung 17 dient der Zufuhr von frischem Lösungsmittel.

Das in Fig. 2 dargestellte Fließschema zeigt nicht die Nebeneinrichtungen, die bei der Durchführung des erfindungsgemäßen Verfahrens zur Anwendung gelangen. Hierbei handelt es sich in erster Linie um Wärmetauscher zur Aufwärmung bzw. Abkühlung der einzelnen Prozeßströme, die Verdampfer bzw. Umlaufkocher am Sumpf der einzelnen Kolonnen sowie die notwendigen Meß- und Regeleinrichtungen. Für die Erläuterung bzw. das Verständnis der erfindungsgemäßen Arbeitsweise spielen diese Nebeneinrichtungen jedoch keine Rolle.

Die Einstellung der Temperatur, mit der die einzelnen Lösungsmittelteilströme in die Extraktivdestillationskolonne eingeleitet werden, erfolgt zweckmäßigerweise dadurch, daß kaltes und wiederaufgeheiztes Lösungsmittel vor dem Eintritt in die Kolonne im entsprechenden Verhältnis miteinander gemischt werden.

Die bei Anwendung des erfindungsgemäßen Verfahrens erzielbaren Vorteile lassen sich wie folgt zusammenfassen:

- Erhöhung der Durchsatzkapazität der Extraktivdestillationskolonne um bis zu 2o %.
- Ruhigere Fahrweise der Extraktivdestillationskolonne. Eine Zweiphasenbildung auf den oberen Böden und damit ein Stoßen der Kolonne findet nicht mehr statt.
- Der Restaromatengehalt in den Nichtaromaten des Raffinates wird nachhaltig gesenkt. Im Falle der Benzolgewinnung aus druckraffiniertem Kokereibenzol sank beispielsweise der Aromatengehalt des Raffinates von 15 auf 3 Gew.-%.
- Die Verunreinigung der Reinaromaten an besonders schwierig zu entfernenden Nichtaromaten, wie z.B. Methylcyclohexan oder bestimmten anderen Naphthenen, ging signifikant zurück.
- Wegen des guten Trennergebnisses in der Extraktivdestillation kann die Fahrweise der Lösungsmittelrückgewinnungskolonne derartig verbessert werden, daß die Lösungsmittelverluste auf einen praktisch nicht mehr nachweisbaren Wert absanken.

Die Wirkungsweise des erfindungsgemäßen Verfahrens wird durch den nachfolgenden Vergleichsversuch belegt.

Vergleichsversuch

Für die Durchführung dieses Vergleichsversuches wurde eine Extraktivdestillationskolonne mit 6o Böden verwendet, wobei die Einsatzproduktaufgabe jeweils auf den 3o. Boden erfolgte. In Teil A des Vergleichsversuches wurde in an sich bekannter Weise die gesamte Lösungsmittelmenge auf den obersten Boden der Extraktivdestillationskolonne aufgegeben. In Teil B des Vergleichsversuches wurden dagegen erfindungsgemäß 37,5 Gew.-% der Gesamtlösungsmittelmenge in zwei Teilströmen aufgeteilt auf Böden oberhalb der Einsatzproduktaufgabe in die Extraktivdestillationskolonne eingeleitet. Selbstverständlich gelangte dabei in beiden Teilen des Vergleichsversuches ein Einsatzprodukt gleicher Zusammensetzung sowie die gleiche Extraktivdestillationskolonne zur Anwendung. Als selektives Lösungsmittel wurde jeweils N-Formylmorpholin (NFM) eingesetzt. Die erzielten Versuchsergebnisse lassen sich wie nachfolgend dargestellt zusammenfassen, wobei sich die Bezugszeichen auf das Fließschema in Fig. 2 beziehen.

4

Teil A:    Einsatz produkt    durch
           Leitung 1:                750 kg/h Benzol
                                     150 kg/h Paraffine
                                     100 kg/h Naphthene
                                    1000 kg/h


           Lösungsmittel
           durch Leitung 3:         4000 kg/h NFM
                                      40 kg/h Benzol
                                    4040 kg/h

Teil A:    Einsatz produkt    durch

Sumpfprodukt
durch Leitung 5:          4000,00 kg/h NFM
                           765,00 kg/h Benzol
                             0,25 kg/h Paraffine
                             0,50 kg/h Naphthene
                          4765,75 kg/h


Kopfprodukt
durch Leitung 4:           149,75 kg/h Paraffine
                            99,50 kg/h Naphthene
                            25,00 kg/h Benzol
                           274,25 kg/h


Teil B:      Einsatzprodukt
             durch Leitung 1:      900 kg/h Benzol
                                   180 kg/h Paraffine
                                   120 kg/h Naphthene
                                  1200 kg/h


             Lösungsmittel
             durch Leitung 3:     3000 kg/h NFM
                                    30 kg/h Benzol


             Lösungsmittel
             durch Leitung 9:     1000 kg/h NFM
                                    10 kg/h Benzol
             (3. Boden oberhalb
             der Einsatzproduktaufgabe)

6

```
Lösungsmittel
durch Leitung 1o:        8oo kg/h NFM
                           8 kg/h Benzol
(5. Boden oberhalb
der Einsatzproduktaufgabe)


Sumpfprodukt
durch Leitung 5:        48oo,oo kg/h NFM
                          936,oo kg/h Benzol
                            o,3o kg/h Paraffine
                            o,4o kg/h Naphthene
                         5736,7o kg/h


Kopfprodukt
durch Leitung 4:         179,7 kg/h Paraffine
                         119,6 kg/h Naphthene
                          12,o kg/h Benzol
                         311,3 kg/h
```

Ein Zahlenvergleich bestätigt die weiter oben geltend gemachten Vorteile des erfindungsgemäßen Verfahrens. So kann bei Anwendung des erfindungsgemäßen Verfahrens trotz Einsatzes der gleichen Extraktivdestillationskolonne die Einsatzproduktaufgabe pro Stunde um 20 % gesteigert werden. Gleichzeitig ging dabei der Benzolgehalt im Kopfprodukt (Raffinat) von 9,12 % auf 3,85 % zurück, während der Nichtaromatengehalt des im Sumpfprodukt (Extrakt) gewonnenen Benzol von 0,098 % auf 0,075 % absank.

## Ansprüche

1. Verfahren zur Abtrennung von Aromaten aus Kohlenwasserstoffgemischen beliebigen Aromatengehaltes durch Extraktivdestillation unter Verwendung von N-substituerten Morpholinen, deren Substituenten nicht mehr als 7 C-Atome aufweisen, als selektivem Lösungsmittel, wobei nur ein Teil der erforderlichen Gesamtlösungsmittelmenge auf den obersten Boden der Extraktivdestillationskolonne aufgegeben wird, dadurch gekennzeichnet, daß der Teil der Gesamtlösungsmittelmenge, der nicht auf den obersten Boden der Extraktivdestillationskolonne aufgegeben wird, zwischen 10 und 40 Gew.-% beträgt, wobei dieser Teil in mindestens zwei Teilströme aufgeteilt und in Abhängigkeit vom Temperaturverlauf in der Extraktivdestillationskolonne auf Böden oberhalb der Einsatzproduktaufgabe in der Weise in die Extraktivdestillationskolonne eingeleitet wird, daß die Menge des über die einzelnen Teilströme zugeführten Lösungsmittels in Richtung auf den obersten Boden von Stufe zu Stufe abnimmt und wobei ferner die Aufgabetemperatur des jeweiligen Teilstromes so eingestellt wird, daß sie die Temperatur auf dem entsprechenden Aufgabeboden nicht übersteigt und auch nicht um mehr als 10 $^\circ$C unterschreitet.

2. Verfahren nach Anspruch 1, dadurch gekennzeichnet, daß der Teil der Gesamtlösungsmittelmenge, der nicht auf den obersten Boden der Extraktivdestillationskolonne aufgegeben wird, vorzugsweise in zwei bis fünf Teilströme aufgeteilt wird.

3. Verfahren nach den Ansprüchen 1 und 2, dadurch gekennzeichnet, daß die Einleitung der einzelnen Teilströme des Lösungsmittels in die Extraktivdestillationskolonne, vorzugsweise zwischen dem dritten und zehnten Boden oberhalb der Einsatzproduktaufgabe erfolgt.

## Claims

1. Process for separating aromatics from hydrocarbon mixtures of any aromatics content by extractive distillation using N-substituted morpholines whose substituents have not more than 7 C atoms as selected solvent, only a part of the total solvent rate required being charged to the top tray of the extractive distillation column, characterised in that the part of the total solvent rate which is not charged to the top tray of the extractive distillation column amounts to between 10 and 40% by weight, this part being divided into at least two part streams and being introduced into the extractive distillation column, as a function of the temperature curve in the extractive distillation column, to trays above the feed product feed point in such a way that the rate of solvent fed via the individual part streams decreases from stage to stage in the direction of the top tray and, moreover, the charging temperature of the particular part stream being adjusted such that it does not exceed the temperature of the corresponding feed tray and also is not more than 10˚C lower.

2. Process according to Claim 1, characterised in that the part of the total solvent rate which is not charged to the top tray the extractive distillation column is preferably divided into two to five part streams.

3. Process according to Claims 1 and 2, characterised in that the individual part streams of the solvent are introduced into the extractive distillation column preferably between the third and tenth tray above the feed product feed point.

## Revendications

1. Procédé pour séparer des composés aromatiques de mélanges d'hydrocarbures comportant des teneurs quelconques en composés aromatiques, moyennant la mise en oeuvre à titre de solvant sélectif de morpholines N-substituées dont les fonctions de substitution ne présentent pas plus de 7 atomes de C, tandis qu'une partie seulement du volume total du solvant nécessaire est délivrée sur le plateau le plus haut de la colonne de distillation extractive, caractérisé par le fait que la partie du volume total de solvant qui n'est pas délivrée sur le plateau le plus haut de la colonne de distillation extractive, fait entre 10 et 40 % en poids, tandis que ladite partie est partagée en au moins deux flux partiels et injectée dans la colonne de distillation extractive dans des plateaux au-dessus du point de chargement du produit à traiter en fonction de l'allure de la température dans la colonne de distillation extractive, de manière tel le que le volume de solvant apporté par le biais des différents flux partiels diminue d'un niveau à l'autre en direction du plateau le plus haut, et tandis qu'en outre la température d'injection de chacun des flux partiels est ajustée de telle sorte qu'elle ne dépasse pas la température sur le plateau d'injection correspondant et ne soit pas non plus inférieure de plus de 10˚C à cette dernière.

2. Procédé suivant la revendication 1,
caractérise par le fait que la partie du volume total de solvant qui n'est pas délivrée sur le plateau le plus haut de la colonne de distillation extractive, est partagée de préférence en deux à cinq flux partiels.

3. Procédé suivant les revendications 1 et 2,
caractérisé par le fait que l'injection des différents flux partiels du solvant dans la colonne de distillation extractive est effectuée de préférence entre le troisième et le dixième plateau -au-dessus du point de chargement du produit à traiter.

Fig. 1

Fig. 2